# EUROPEAN PATENT SPECIFICATION

(11) **EP 0 800 696 B1**
(45) Date of publication and mention of the grant of the patent: **27.01.1999**
(21) Application number: 95942783.2
(22) Date of filing: 20.12.1995
(51) Int. Cl.: G09B 21/00, A61F 4/00

(54) **A SYSTEM FOR COMMUNICATION BETWEEN A HANDICAPPED PERSON AND HIS SURROUNDINGS**
SYSTEM ZUR KOMMUNIKATION ZWISCHEN EINER BEHINDERTEN PERSON UND IHRER UMGEBUNG
SYSTEME DE COMMUNICATION ENTRE UNE PERSONNE HANDICAPEE ET SON ENVIRONNEMENT

(30) Priority: 23.12.1994 NL 9402199
(43) Date of publication of application: 15.10.1997
(73) Proprietor: EURO EAR B.V., 1059 BW Amsterdam (NL)
(72) Inventor: OOMS, Simon, Frederik, NL-6881 MJ Velp (NL)
(74) Representative: Louet Feisser, Arnold
(86) International application number: NL9500430
(87) International publication number: WO9620466

(56) References cited:
- WO-A-87/01229
- DE-A- 4 033 673
- FR-A- 2 660 467
- GB-A- 2 041 599
- GB-A- 2 138 616
- GB-A- 2 221 070
- US-A- 4 768 926

## Description

The invention relates to a system for communication between a handicapped person and his surroundings, whereby the handicapped person carries a transmitter and/or receiver which is capable of wireless communication with a receiver and/or transmitter present at a fixed location in the handicapped person's surroundings. GB-A-2 138 616, which represents the closest prior art, discloses such a system according to the preamble of claim 1.

A system of this kind can for example be used by a deaf person who is carrying a vibrating receiver. A vibrating receiver is a device which can readily be carried on or underneath a person's clothes and which can vibrate in such a manner that the deaf person can recognize this as a signal. The vibrating of the receiver is thereby caused by the signal which is wirelessly transmitted to the receiver from a transmitter which is present at a fixed location in the deaf person's surroundings. Such a signal may be sent when a sound source produces a sound, for example when the doorbell goes, the telephone rings, a baby cries, etc. The transmitter is thereby wirelessly or wire-connected to the various sound sources. When the sound source is a doorbell or a telephone, an electric signal can be tapped directly from such a source for that purpose, whilst a microphone may be used to detect the crying of a baby. Depending on the sound source in question the transmitter may transmit a specific code to the receiver, so that the deaf person can identify the sound source in question from the manner in which the receiver vibrates or from other visual signals to be delivered by the receiver. A system of this kind was marketed under the name of "VisaBel" in the Netherlands in 1995, by the firm of Goedhard Amersfoort.

Another known system was marketed under the name of "Lisa", among others by Auditech B.V. A number of transmitters is thereby installed in a deaf person's house. Each transmitter can be plugged into a wall socket of the electric mains and be connected to the sound source by means of an electric wire, whereby sound being produced at that location can be detected by means of a microphone or by tapping an electric signal. Each transmitter can transmit a code characteristic of that particular transmitter to a number of receivers, which, depending on the code, all deliver a particular light flash or a series of light flashes. A receiver present in the bedroom may thereby be wire-connected to a vibrating alarm, which may for example be placed under the pillow.

Another system is known because it was introduced on the market for testing under the name of "Danalert" by Danavox Nederland B.V. This system likewise comprised several transmitters, which each transmit a particular code to a receiver carried on a person's body when the transmitter receives a particular signal.

A drawback of the known systems is among other things the fact that they are relatively expensive, because a great deal of different components are required, whilst in addition to that the installation of such a system is complicated, because the transmitter must somehow be connected to the various sound sources.

The object of the invention is to provide a system for communication between a handicapped person and his surroundings, which system is multifunctional, has relatively few components and which can be readily installed in a house.

According to one aspect of the invention the system is provided with one or more transmitters, which comprise means for manually setting the code to be transmitted. If a vibrating receiver carried by a deaf person must be capable of receiving signals from various sound sources, it is possible in this manner to place an identical transmitter near each sound source, which transmits a signal received at the location of said source to said vibrating receiver, and that by means of a code pre-tuned to that particular receiver, which code may be selected by the deaf person himself. Since the transmitters present at the various sound sources are each tuned to a different code, they can all transmit to the same receiver, whereby the receiver will reproduce the code received. The deaf person is able to tell from the code in question, for example four lights on the receiver lighting up in various combinations thereof, which sound source is delivering a signal.

The multifunctional system according to the invention may also be used for the remote control of devices and installations by a handicapped person. A number of identical receivers are thereby placed near installations to be controlled, for example for opening or closing a door or a window, for turning a television on or off, etcetera. The handicapped person thereby carries a transmitter, whereby he can set the code to be transmitted by said transmitter. The code set in this manner is then transmitted to all receivers, and one or several of the control devices near which the receivers are placed are actuated on the basis of the code transmitted. A control device may be a stepping motor, which opens or closes a window or a door, or an electric switch, which turns an electric apparatus on or off.

According to one aspect of the invention also the transmitters may be provided with the same visual indication means as the receivers. This makes it possible to see the code on the transmitter when said code is being set. Furthermore this makes it possible to use a substantially identical housing for the transmitter(s) and the receiver(s), which has a cost-reducing effect.

According to the invention a receiver, hereinafter called a primary receiver, may furthermore be provided with a secondary transmitter, which is capable of wirelessly transmitting a signal to a secondary receiver. If the primary receiver is provided with a vibrating mechanism, it may be desirable for a person to carry said receiver underneath his clothes. In that case said person may for example wear a wristband containing a small secondary receiver, on which the visual signal is displayed. Since the secondary transmitter/receiver only needs to bridge a small distance, the components in question may be small.

A secondary receiver may also be fitted with a special vibrating mechanism, which may for example be placed under the pillow during the night, so that a deaf person can be woken up by said mechanism. In the meantime the primary receiver may be placed in a recharging device, so that a rechargeable battery can be recharged during the night. During the day the special vibrating mechanism may be placed in the same recharging apparatus in order to be recharged.

A major advantage of a wireless vibrating mechanism capable of waking up a deaf person during the night is the absence of a feed wire, which may constitute a danger in bed.

According to another aspect of the invention the transmitter is adapted to transmit a warning signal to a receiver being in communication with said transmitter when the battery of the transmitter is running down. Since the transmitters of the multifunctional system according to the invention may be installed at several locations, there is thus no need to be afraid that the running down of a battery in a transmitter being located somewhere in the handicapped person's house will go unnoticed.

According to one aspect of the invention one or more transmitters and/or one or more receivers are fitted with a multipolar terminal, for example a plug connection comprising six electric conductors, by means of which the transmitter and/or the receiver can be connected to a signalling device. The term signalling device is understood to include a microphone, a telephone or a doorbell, which deliver a signal to a transmitter, as well as a control device which receives a signal from the receiver.

A six-polar plug can be connected to the transmitter or receiver by means of a multipolar terminal comprising six conductors, to which plug a two-conductor or multi-conductor conducting wire to a doorbell or a telephone or a microphone may be connected, for example. By connecting the conducting wire to several poles of the plug, depending on the voltage or the nature of the electric signal, the transmitter may be adapted to process the signal into a code to be transmitted. The form of the code can be set as desired on each of the transmitters and be recognized as such by the receiver.

When a number of control devices are to be controlled by means of one transmitter by a handicapped person, each control device may be connected to two or more poles of the receiver in question, whereby particular poles of the connection to all receivers are excited on the transmitter by setting the code, whereby the control device connected to the poles in question can be actuated in this manner.

According to a further aspect of the invention the transmitters and the receivers have an identical housing, which will lead to a further saving on production costs. The transmitters and the receivers may thereby be provided with identical inputs and outputs, such as the aforesaid multipolar terminal, signalling lights, for example four, a means for attaching the transmitters and receivers to a handicapped person or to a fixed object, and a terminal for recharging a rechargeable battery present in the transmitter or the receiver, so that they can all be placed in the same recharging device.

The invention furthermore relates to a method of communication, said method functioning to alert a person or to control devices by remote control or to deliver or receive signals, utilizing the above-described system.

Further aspects of the invention, which may be used separately or in combination with each other, will be described with reference to the Figures and be defined in the claims.
Figure 1 is a schematic view of the system to be used for alerting a deaf person;
Figure 2 schematically shows the system used as a remote control system;
Figure 3 schematically shows the components of a portable receiver;
Figures 4 - 8 are views of a transmitter/receiver; and
Figure 9 is a perspective view of a transmitter.

Like parts are numbered alike in the schematic Figures.

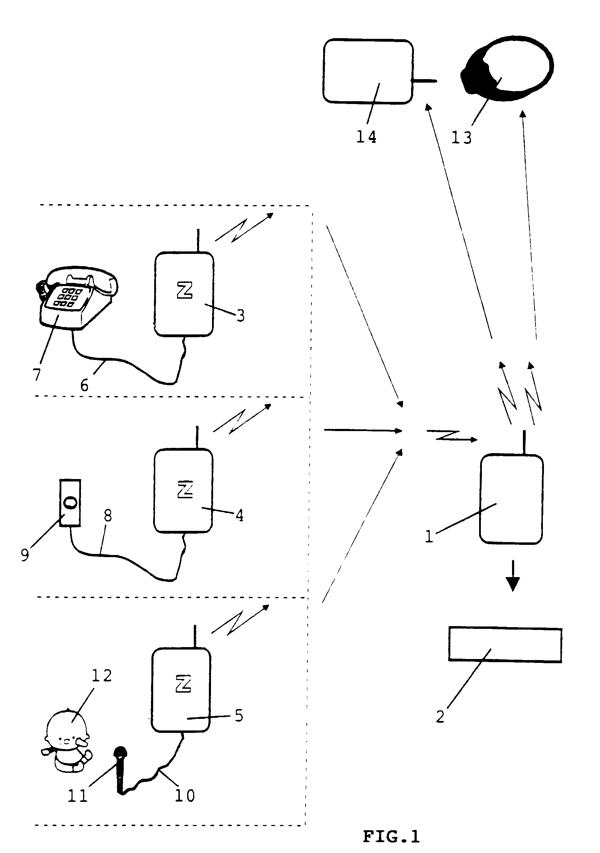

Figure 1 shows a portable receiver 1, which is provided with a vibrating mechanism and which can be carried by a deaf person in such a manner that said person can be alerted by vibrations of the receiver 1. To that end receiver 1 is for example carried on a person's body, underneath his clothes. Receiver 1 is provided with a rechargeable battery and may be placed in recharging device 2 for recharging of the battery, which recharging device may be connected to the mains via an adaptor. Receiver 1 may be placed in recharging device 2 during the night, for example.

Portable receiver 1 may receive signals from several transmitters 3, 4, 5, three of which are shown. Transmitter 3 is connected to telephone 7 by means of a cord, in such a manner that transmitter 3 is able to detect the ringing of the telephone. To that end cord 6 may for example be connected to the plug by which telephone 7 is connected to the telephone network. Cord 6 may furthermore be provided with a magnetic or other type of receiver.

Transmitter 4 is connected to doorbell 9 by means of a cord 8. Also cord 8 may have a special provision for connection to the bell, for example to terminals for tapping an electric signal from the doorbell.

Transmitter 5 is connected, by means of cord 10, to a microphone 11, which is capable of detecting the crying of a baby 12, for example.

Cords 6, 8, 10 may each be connected, by means of identical multipolar plugs, to transmitters 3, 4, 5, whereby each of the cords 6, 8, 10 may utilize specific poles of the multipolar, for example sixpolar plug. This makes it possible for a cord 6, 8, 10 to be made specifically for a specific application, whereby the correct input of the transmitter 3, 4, 5 is used at all times. As is indicated by arrows in Figure 1, the transmitters can transmit the result of their detections to portable receiver 1, which will start to vibrate as a result of such a signal. The vibration may thereby be such that the origin of the signal can be recognized.

Each of the transmitters 3, 4, 5 may be tuned to transmit a specific code. This enables the receiver 1 to recognize which transmitter is transmitting a signal, in spite of the fact that transmitters 3, 4, and 5 are identical. The manner in which the transmitters are to be tuned will be explained hereafter in more detail.

Receiver 1 not only comprises a receiver, but also a secondary transmitter, which is capable of wirelessly transmitting signals to a secondary receiver. Said secondary receiver may be provided in a wristband 13 and/or a pillow vibrator 14. If a person carries the receiver 1 underneath his clothes, the code may be displayed by means of the secondary wristband receiver 13 in such a manner that the origin of the signal can be established visually. This may take place by means of lights or in a different manner. The pillow vibrator 14 is a device which looks the same as the receiver 1, it may be put under the pillow during the night, for example, in order to perform a waking function when a signal is transmitted. Since the pillow vibrator is cordless, it may be placed in a great many different places in a bed without this constituting a danger.

Pillow vibrator 14 is likewise provided with a rechargeable battery and has a shape which corresponds with that of receiver 1 in such a manner that the recharging of the battery of pillow vibrator 14 can take place in recharging device 2. Thus receiver 1 may be placed in the recharging device 2 during the night, whereby signals can be transmitted to vibrator 14, whilst vibrator 14 can be placed in the same recharging device 2 during the day.

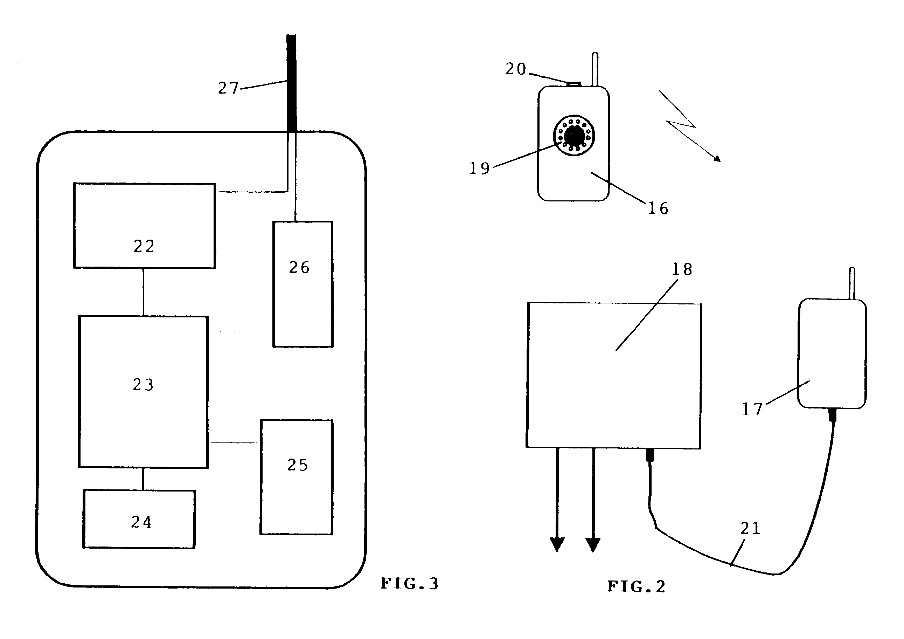

Figure 2 diagrammatically shows a system wherein a transmitter 15 and a receiver 17 are used for activating a signalling device 18 by remote control. Transmitter 16 is provided with a selector switch in the shape of a dial 19, and with a starter button 20, which can be depressed in order to transmit the code, which is determined by the position of dial 19, to receiver 17. Depending on the code that has been set, receiver 18 will transmit, via cord 21, a signal to signalling device 18, which, depending on the code, will actuate control equipment. This may for example be electric, electro-pneumatic or electro-hydraulic control equipment, for example for operating doors, lifts, lighting equipment, etc. Receiver 17 may be tuned to receive a single, specific code and, after recognizing said code, may transmit said code to signalling device 18, which subsequently carries out a particular operation. Receiver 17 may also be tuned to receive several different codes, however, and transmit a specific signal to signalling device 18 in dependence on said code, which signalling device will carry out a particular operation, depending on the code in question. In that case cord 21 may be a multiconductor cord, whereby transmitter 17 transmits a signal to signalling device 18 via particular conductors of said cord, depending on said code.

The system shown in Figure 2 may also comprise several receivers 17, which each react to the particular code set thereon, so that it is possible to determine by means of selector switch 19 which receiver will be activated.

Figure 3 schematically shows the components which may be present in a receiver. These components are: a radio receiver 22, a logic circuit 23, a rechargeable battery 24, a vibrator 25, a secondary transmitter 26 and an antenna 27. Other possible components, such as a pushbutton, a display or lights and a connecting plug are not shown in Figure 3.

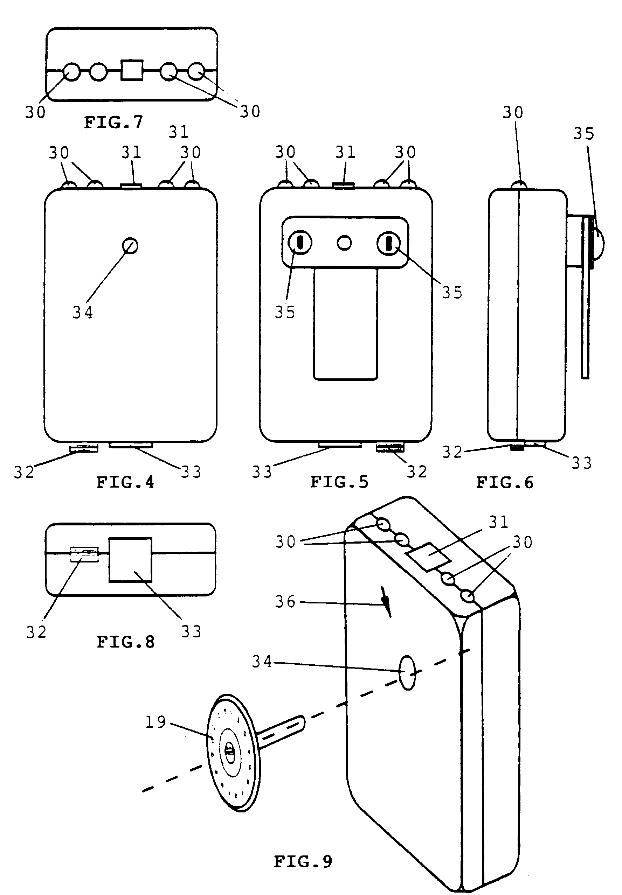

Figures 4 - 8 are views of a universal transmitter and/or receiver. Both the transmitter and the receiver look substantially the same, with a standard housing being used, which may also be used for the pillow vibrator. The transmitter and/or receiver is provided with four lights 30, which, in combination with each other, may display a large number of visually recognizable codes, with a pushbutton 41, by means of which a signal may be transmitted in case a portable transmitter is being used, and by means of which it may be checked which code is set if a stationary transmitter is being used, with an on/off switch 32 for turning the transmitter and/or receiver on and off, with a universal multipolar, for example sixpolar, connecting plug, to which a cord may be connected for transmitting a signal, with an aperture 34 for setting a code to be transmitted if a transmitter is concerned, and witch fastening means comprising two screws 35, which screws are connected to the rechargeable battery within the transmitter and/or receiver in such a manner that said screws serve as conductors during the recharging of the battery.

The fastening means of Figures 5 and 6 consist of a clip, so that the transmitter and/or receiver are easy to carry.

Figure 9 is a perspective view of a transmitter, whereby a dial 19 extends through the aperture 34, by means of which dial a code to be transmitted can be set by turning a particular number to a position opposite indication arrow 36. The transmitter according to Figure 9 may be used for remote control, whereby a signal is transferred by pressing button 31 after a particular code has been set by means of dial 19, whereby the signal may be checked visually in that the lights 30 will light up in a particular combination thereof.

By means of the embodiment a system has been described which can be used universally for several functions, whereby identical or similar components are used in every case. In particular it is possible thereby to use identical housings for a large number of components, which is advantageous from an economic point of view, but which is also advantageous for other reasons, as has been described above.

A standard version of the system for example consists of two universal transmitters, one receiver, a recharging device and a number of specific connecting cords, such as for a telephone and for a doorbell. The numbers of transmitters may be extended as desired with identical transmitters, which are set to a particular code.

With transmitters which are disposed at a fixed location, for example near the doorbell and near the telephone, this code may for example be set through aperture 34 by means of a screwdriver, after which the code can be checked by pressing pushbutton 31. This test button will also activate the transmitter, so that its operation can be checked.

## Claims

1. A system for communication between a handicapped person and his surroundings, whereby the handicapped person carries a receiver (1) which is capable of wireless communication with at least two transmitters (3,4,5) present at fixed locations in the handicapped person's surroundings, whereby each transmitter (3,4,5), when it is activated, wirelessly transmits a predetermined code, and whereby this predetermined code is received by the receiver (1), which indicates which code was transmitted, characterized in that the code to be transmitted by the transmitter (3,4,5) can be preselected manually on that transmitter (3,4,5) by the handicapped person.

2. A system according to claim 1, characterized in that the receiver (1) includes a transmitter for transmitting signals to one or more receivers present at fixed locatons in the handicapped person's surroundings.

3. A system according to any of the preceding claims, wherein the receiver (1) is provided with visual indication means (30) to indicate a particular code, which code is wirelessly transmitted to the receiver (1) by a transmitter (3,4,5), characterized in that the transmitter (3,4,5) is provided with corresponding visual indication means (30), which are capable of displaying said code when said code is being set.

4. A system according to any one of the preceding claims, characterized in that a receiver (1), herein after called primary receiver, is provided with a secondary transmitter, which is capable of wirelessly transmitting a signal to a secondary receiver.

5. A system according to claim 4, characterized in that said secondary receiver comprises a vibrating mechanism (14).

6. A system according to claim 5, characterized in that said secondary receiver is suitable to be placed in the same recharging device when the primary receiver (1) is not present therein.

7. A system according to any one of the preceding claims, characterized in that said transmitter (3,4,5) sends a warning signal to a receiver (1) when the battery of said transmitter (3,4,5) has nearly run down.

8. A system according to any one of the preceding claims, characterized in that said transmitters (3,4,5) and said receivers (1) have substantially identical housings.

## Patentansprüche

1. Ein System zur Kommunikation zwischen einer behinderten Person und ihren Umgebungen, wozu die behinderte Person einen Empfänger (1) trägt, der zur drahtlosen Kommunikation mit wenigstens zwei Sendern (3,4,5) geeignet ist, die sich an festen Orten in der Umgebung der behinderten Person befinden, wobei jeder Sender (3,4,5), wenn er aktiviert ist, einen bestimmten Code drahtlos überträgt, und wobei der bestimmte Code vom Empfänger (1) empfangen wird, der anzeigt, welcher Code gesendet wurde, dadurch gekennzeichnet, daß der durch den Sender (3,4,5) auszusendende Code durch die behinderte Person an dem Sender (3,4,5) manuell im voraus ausgewählt werden kann.

2. Ein System gemäß Anspruch 1, dadurch gekennzeichnet, daß der Empfänger (1) einen Sender zum Senden von Signalen zu einem oder mehreren Empfängern enthält, die sich an festen Orten in den Umgebungen der behinderten Person befinden.

3. Ein System gemäß einem der vorausgehenden Ansprüche, worin der Empfänger (1) mit visuellen Anzeigemitteln (30) versehen ist zur Anzeige eines bestimmten Codes, der durch einen Sender (3,4,5) drahtlos zu dem Empfänger (1) übertragen wird, dadurch gekennzeichnet, daß der Sender (3,4,5) mit visuellen Anzeigemitteln (30) ausgestattet ist, die geeignet sind, den Code anzuzeigen, nachdem er eingestellt worden ist.

4. Ein System gemäß einem der vorausgehenden Ansprüche, dadurch gekennzeichnet, daß ein Empfänger (1), der nachfolgend als primärer Empfänger bezeichnet wird, mit einem sekundären Sender ausgestattet ist, der zu einer drahtlosen Übertragung eines Signals zu einem sekundären Empfänger geeignet ist.

5. Ein System gemäß einem der vorausgehenden Ansprüche, dadurch gekennzeichnet, daß besagter sekundärer Empfänger einen Vibrationsmechanismus (14) umfaßt.

6. Ein System gemäß einem der vorausgehenden Ansprüche, dadurch gekennzeichnet, daß besagter sekundärer Empfänger so gestaltet ist, daß er in dieselbe Wiederaufladevorrichtung eingesetzt werden kann, wenn sich der primäre Empfänger (1) nicht darin befindet.

7. Ein System gemäß einem der vorausgehenden Ansprüche, dadurch gekennzeichnet, daß besagter Sender (3,4,5) ein Warnsignal an einen Empfänger (1) sendet, wenn die Batterie des besagten Senders (3,4,5) nahezu entladen ist.

8. Ein System gemäß einem der vorausgehenden Ansprüche, dadurch gekennzeichnet, daß besagter Sender (3,4,5) und besagter Empfänger (1) im wesentlichen identische Gehäuse aufweisen.

## Revendications

1. Système de communication entre une personne handicapée et son environnement, dans lequel la personne handicapée porte un récepteur (1) qui est susceptible de communiquer sans fil avec au moins deux émetteurs (3, 4, 5) qui sont situés en des emplacements fixes de l'environnement de la personne handicapée, dans lequel chaque émetteur (3, 4, 5), lorsqu'il est activé, transmet par voie sans fil un code prédéterminé, et dans lequel ce code prédéterminé est reçu par le récepteur (1) qui indique quel code a été transmis, caractérisé en ce que le code à transmettre par l'émetteur (3, 4, 5) peut être présélectionné manuellement sur cet émetteur (3, 4, 5) par la personne handicapée.

2. Système selon la revendication 1, caractérisé en ce que le récepteur (1) comprend un émetteur pour transmettre des signaux à un ou plusieurs récepteurs situés en des emplacements fixes de l'environnement de la personne handicapée.

3. Système selon l'une quelconque des revendications précédentes, dans lequel le récepteur (1) comporte des moyens d'indication visuelle (30) pour indiquer un code particulier, lequel code est transmis par voie sans fil au récepteur (1) par un émetteur (3, 4, 5), caractérisé en ce que l'émetteur (3, 4, 5) comporte des moyens d'indication visuelle (30) correspondants qui sont susceptibles d'afficher ledit code, lorsque ledit code va être choisi.

4. Système selon l'une quelconque des revendications précédentes, caractérisé en ce qu'un récepteur (1), ci-après dénommé récepteur primaire, comporte un émetteur secondaire, qui est capable de transmettre par voie sans fil un signal à un récepteur secondaire.

5. Système selon la revendication 4, caractérisé en ce que ledit récepteur secondaire comprend un mécanisme vibrant (14).

6. Système selon la revendication 5, caractérisé en ce que ledit récepteur secondaire est conçu de façon à être placé dans le même dispositif de recharge, lorsque le récepteur primaire (1) n'y est pas présent.

7. Système selon l'une quelconque des revendications précédentes, caractérisé en ce que ledit émetteur (3, 4, 5) envoie un signal d'alarme à un récepteur (1), lorsque la batterie dudit émetteur (3, 4, 5) est proche de la décharge.

8. Système selon l'une quelconque des revendications précédentes, caractérisé en ce que lesdits émetteurs (3, 4, 5) et lesdits récepteurs (1) ont des boîtiers sensiblement identiques.
